# EUROPEAN PATENT APPLICATION

(11) **EP 4 210 072 A1**
(43) Date of publication of application: **12.07.2023**
(21) Application number: 22150521.7
(22) Date of filing: 07.01.2022
(51) Int. Cl.: G16H 50/30, G16H 50/70, A61B 5/00, A61B 5/055

(54) **ANALYSIS OF COGNITIVE IMPAIRMENT USING MRI**

(71) Applicant: Koninklijke Philips N.V., 5656 AG Eindhoven (NL)
(72) Inventor: KLAMING, Laura, Eindhoven (NL); BEKKER, Evelijne Machteld, Eindhoven (NL); VAN EE, Raymond, Eindhoven (NL); MENA BENITO, Maria Estrella, Eindhoven (NL); VAN DOOREN, Marieke, Eindhoven (NL)
(74) Representative: Philips Intellectual Property & Standards

(57) **Abstract**

A method and apparatus for use as part of diagnostic analysis of the psychiatric or neurological health of a patient based on use of a normative database of fMRI data results and cognitive task results for a population of prior patients. Embodiments propose to acquire fMRI data of a particular set of one or more regions of the brain of a patient while the patient is performing a cognitive task, and to record at the same time results of the patient's performance in the cognitive task. A combination of both the cognitive task results and the fMRI data for the brain regions is used to provide an indicator as to a cognitive health status of the patient. More particularly, a normative database of fMRI data results and cognitive task results for a population of patients is used as a reference. This may store results both for healthy patients and pathological patients, to provide a normal and abnormal reference.

## Description

### FIELD OF THE INVENTION

The present invention relates to a device for assisting diagnosis of neurological disorders using MRI data.

### BACKGROUND OF THE INVENTION

Magnetic resonance imaging (MRI) scanners rely on a large static magnetic field (Bo) to align the nuclear spins of atoms as part of the procedure for producing images within the body of a patient. These images can reflect various quantities or properties of the subject. For example, the hemodynamic response of brain activation causes magnetic and electric changes in the activated brain area.

In cerebral imaging, MRI can be used to image the structure of the brain or to image the functional activity of the brain. The latter is known as functional MRI (fMRI). Functional MRI measures brain activity by magnetically detecting variations in local areas associated with blood flow. Cerebral blood flow and neuronal activation are coupled, and therefore the changing blood flow in a local area indicates brain activity in that area. In other words, when an area of the brain is in use, blood flow to that region also increases.

There are different specific metrics of neural activation that can be derived from fMRI data. The primary form of fMRI uses the blood-oxygen-level dependent (BOLD) contrast imaging. Other approaches include arterial spin labeling and diffusion MRI. The latter procedure is similar to BOLD fMRI but provides contrast based on the magnitude of diffusion of water molecules in the brain.

With regards to BOLD contrast imaging, this is based on detecting differences in magnetic properties between arterial (oxygen-rich) and venous (oxygen-poor) blood. When neurons in a given brain region become active, local blood flow in the region increases, and, after a certain delay of around two seconds, oxygen-rich (oxygenated) blood starts to displace oxygen-depleted (deoxygenated) blood. The blood flow continues to increase over a 4-6 second period, rising to a peak, and subsequently falling back to a lower level, at or slightly below the original level. Deoxygenated hemoglobin (dHb) is more magnetic (paramagnetic) than oxygenated hemoglobin (Hb), the latter of which is effectively resistant to magnetism (diamagnetic). This difference leads to an improved MR signal from the oxygenated brain regions, since the diamagnetic blood in the oxygenated regions interferes with the magnetic MR signal less. This improvement can be mapped as a function of position in the brain and as a function of time, to map which neurons are active at any given time.

Functional MRI can be used as an investigative tool within the field of neurological illness and psychiatric disorder, and associated brain injury. This can involve administering one or more cognitive tasks or tests to a patient, which are known to activate specific areas of the brain in healthy subjects in specific patterns.

Within the field of neurological illness and psychiatric illness, it is known to use cognitive tests or tasks as diagnostic tools in differentially diagnosing a psychiatric disorder.

However, impaired performance in a given task may be associated with multiple different psychiatric disorders, or may be associated with physical brain injury. In the latter regard, impaired performance on a given cognitive test or task can be caused by potential impairment at various different locations of the brain and/or various types of acquired or neurodegenerative brain damage, e.g. traumatic brain injury (TBI), cerebrovascular accidents (CVA), mild cognitive impairment (MCI) or Alzheimer's disease (AD). Psychiatric disorders can also be associated with impaired performance in certain cognitive tasks, even in the absence of any physical brain injury.

It is furthermore known that brain lesions in different brain locations can present similar results on cognitive tests. Similarly, it is known that brain lesions in the same location can cause presentation of different results in cognitive tests. In this regard, reference is made for example to the paper: Darby, R.R., Joutsa, J., & Fox, M.D. (2019). Network localization of heterogeneous neuroimaging findings. Brain, 142(1), 70-79.

In addition, a cognitive test typically involves various cognitive functions, i.e. performing a cognitive test requires the recruitment of different cognitive functions. In fact, if a certain cognitive function is impaired as a result of brain damage, the individual may still perform the cognitive test within the norm because she is able to compensate for the deficit. As a consequence, the result of a cognitive test can be the same for two people with very different underlying deficits and different causes of these deficits, simply because they utilized different underlying cognitive functions and networks to solve the task. This is particularly relevant in mild cases of brain disease such as mild TBI (which accounts for 85% of all TBIs) and mild cognitive impairment (MCI) which is considered a precursor condition of dementia. As a consequence, the outcome of a cognitive test (battery) does not specifically reflect underlying brain damage that causes cognitive deficits. This can lead to misdiagnosis and underdiagnosis of cognitive impairment.

It is known to use MRI imaging as a further diagnostic tool in differentially diagnosing psychiatric disorder and/or brain injury.

Typically, structural MRI imaging is used to detect or rule out brain injury. However, this may not be able to detect injury with subtle structural features. It is also unable to detect neural impairment caused by an injury which has no associated structural presentation.

It is known to use functional MRI imaging to assess a patient's neural activity at a local level as a further diagnostic tool in investigating brain injury and psychiatric illness. However, the results can be difficult to interpret since whole patterns of brain areas may typically activate during performance of a task and it is difficult to differentially determine from this alone what may be causing a presentation of symptoms.

### SUMMARY OF THE INVENTION

The invention is defined by the claims.

According to examples in accordance with an aspect of the invention, there is provided a processing arrangement comprising one or more processors adapted to:
obtain functional MRI (fMRI) data for a patient corresponding to one or more brain areas, wherein the fMRI data is data acquired while the patient is performing a pre-determined cognitive task;
obtain cognitive task results data for the patient, corresponding to the cognitive task administered while acquiring the fMRI data;
derive from the obtained fMRI data neurological activity data;
retrieve from a datastore a pre-acquired database storing reference neurological activity data and reference cognitive task results data associated with a pre-determined neurological or psychiatric disorder of interest and associated with the pre-determined cognitive task, for a population of people (e.g. prior patients);
compare the patient's neurological activity data and cognitive task results data with the reference activity data and reference cognitive task results data; and
provide an output to a user interface based on the comparison.

Embodiments of the present invention propose to use a combination of cognitive task results for a patient, and localized fMRI-derived neural activity data to provide an output to aid in diagnosis of neurological or psychiatric disorders.

This approach involves a novel data analysis step which is not currently executed in clinical practice. This step involves the comparison of a patient's fMRI-derived neurological activity data and/or brain image data during the performance of a cognitive task with an electronic normative database of prior patients performing the same task.

Furthermore, it is not currently known to use a combination of fMRI data and cognitive tasks results data to provide more specific analysis of the patient's condition. This solves a clinical need by improving the accuracy of diagnosis of neurological or psychiatric disorders as well as a wide range of types of acquired and neurodegenerative brain damage and associated cognitive impairment. As a result, improved diagnosis can be expected. This could be used in practice to facilitate more personalized treatment as well as personalized prediction of progression of cognitive impairment (and associated risk factors) over time.

In some embodiments, the fMRI data may be obtained for one or more pre-determined brain areas, wherein the brain areas are associated with a pre-determined neurological or psychiatric disorder of interest. The derived neurological activity data may be activity data associated with each of the one or more brain areas.

The predetermined brain areas referred to above may correspond to pre-determined neural networks within the brain associated with the neurological or psychiatric disorder of interest.

The cognitive task results data are acquired simultaneously with the acquiring of the fMRI data, based on patient responses during administering of the cognitive task.

In some embodiments, the processing arrangement is further adapted to derive a diagnostic indicator for the neurological or psychiatric disorder based on the comparison, and to provide the diagnostic indicator as the output to the user interface. This may be based on applying an algorithm to derive the indicator. It may be based on applying a machine learning algorithm.

In some embodiments, the output to the user interface is indicative of the results of the comparison.

In some embodiments, the neurological activity data is blood-oxygen-level dependent (BOLD) contrast data.

An aim according to at least one set of embodiments is to determine a type, location, and severity of any cognitive impairment and/or associated brain injury (if any). This could be achieved by providing in the datastore a plurality of different databases for different neurological or psychiatric disorders and potentially also for different cognitive tasks, and comparing the acquired data with the reference data in each of these databases.

In some embodiments, the datastore may store a plurality of different databases, each respectively storing reference activity data and reference cognitive task results data associated with a different specific combination of a neurological or psychiatric disorder of interest and a cognitive task administered.

The processing arrangement may query the datastore according to the neurological or psychiatric disorder of interest and the cognitive task to which the newly obtained results data corresponds.

The process may include:
selecting (e.g. an operator selecting) a neurological or psychiatric disorder of interest from a pre-determined set of different disorders;
further selecting, for the selected disorder, one of a set of one or more cognitive tests/tasks which are associated with the selected disorder;
identifying (e.g. using a lookup table or other reference) a set of one or more brain regions of interest associated with the disorder;
acquiring fMRI data associated with those brain regions while administering the selected test/task to the patient;
acquiring, simultaneously with the acquiring of the fMRI data, results of the cognitive task based on subject indications/responses during the task; and
retrieving from the datastore a database storing reference neurological activity data and cognitive task results data for the selected disorder and the selected cognitive task.

In one particular set of embodiments, the processing arrangement may be adapted to apply multiple different cognitive tasks for probing a same single cognitive disorder. For example, the processing arrangement may be adapted to perform the following combination of steps:
obtaining a plurality of fMRI data sets for the patient, each acquired while a different cognitive task is administered to the patient,
obtaining cognitive task results for the patient for each administered cognitive task;
retrieving from the datastore a database associated with each different cognitive task, and further associated with the neurological or psychiatric disorder;
comparing, for each cognitive task, activity data derived from the corresponding fMRI data set with reference activity data in the corresponding retrieved database, and comparing the cognitive task results with the reference cognitive task results data in said corresponding database.

In other words, multiple cognitive tasks are administered and the results compared with reference data, in order to more reliably determine whether or not the patient is suffering from the neurological or psychiatric disorder of interest.

The method may further comprise providing a differential diagnostic indicator based on the above process.

In some embodiments, the database may comprise at least a first database portion which contains data associated with a first population of people (e.g. prior patients) who do not suffer from the neurological or psychiatric disorder of interest, and a second database portion which contains data associated with a second population of people who do suffer from the neurological or psychiatric disorder. The processing arrangement may be adapted to compare the derived neurological activity data and obtained task results data with the data in both the first and second database portions.

The reference neurological activity data referred to in this disclosure may comprise brain activity maps for one or more areas of the brain for the population of people to whom the reference data corresponds. The reference neurological activity data may additionally or alternatively comprise statistical summary data derived from the neurological activity data for the population of people. In addition to the reference neurological activity data, the database may additionally comprise brain image data for the population, and/or may comprise statistical summary data derived from brain image data for the population, e.g. one or more average brain images for the population.

The reference neurological activity data and reference task results data may comprise statistical data pre-computed from activity data and task results data for a population of prior patients. The statistical data may comprise one or more statistical metrics such as an average, median, inter-quartile range, or any other statistical metric.

In some embodiments, the processing arrangement may be adapted to update the database based on the obtained neurological activity data for the patient, and the task results data for the patient.

This may comprise simply appending the obtained neurological activity data and cognitive task results data to the database or may further comprise re-computing statistical data stored by the database, such as updating an average or other statistical metric.

In some embodiments, the data in the database may be stratified based on one or more demographic classifications of the population of people to whom the data pertain, for example based on gender, age, and/or other predefined characteristics.

The processing arrangement may be further adapted to obtain an indication of the one or more demographic classifications for the patient under examination; and to filter the retrieved database according to the demographic classification of the patient before performing the comparison.

In some embodiments, the processing arrangement may be adapted to control administration of a series of cognitive tasks and obtaining fMRI associated with each one. A decision tree structure may be followed, wherein the results of each cognitive task determine which, if any, further task should be administered, for example with an aim to facilitate a narrowing down of the patient diagnosis.

Accordingly, in some embodiments, the processing arrangement may be further adapted to apply a selection algorithm to determine a new cognitive test to be administered to the patient in dependence upon the obtained cognitive task results and/or in dependence upon the obtained neurological activity data.

The processing arrangement may be further adapted to retrieve from the datastore a normative database containing reference activity data and reference cognitive task results data associated with the selected cognitive task.

The processing arrangement may be further adapted to generate control signals for controlling a patient interface control module to administer the selected cognitive task and for controlling an MRI imaging apparatus to obtain second fMRI data of the patient while the selected cognitive task is being administered.

In accordance with some embodiments, the obtaining of the fMRI data may comprise the processing arrangement generating a control signal for controlling an MRI imaging apparatus to acquire the fMRI data. In other embodiments, the processing arrangement may simply passively receive the fMRI data without controlling its acquisition.

A further aspect of the invention provides a system comprising a processing arrangement in accordance with any embodiment described in this disclosure, and an MRI imaging apparatus operatively coupled with the processing arrangement for acquiring fMRI data of the patient.

Another aspect of the invention provides a computer-implemented method comprising the following steps:
obtaining functional MRI (fMRI) data for a patient corresponding to one or more pre-determined areas of the brain, wherein the fMRI data is data acquired while the patient is performing a pre-determined cognitive task;
obtaining cognitive task results data for the patient, corresponding to the task administered while acquiring the fMRI data;
deriving from the acquired fMRI data neurological activity data;
retrieving from a datastore a pre-acquired normative database storing reference neurological activity data and reference cognitive task results data associated with a neurological or psychiatric disorder of interest and with the pre-determined cognitive task, for a population of previous patients;
comparing the patient's neurological activity data and task results data with the reference neurological activity data and reference task results data; and
providing an output to a user interface based on the comparison.

Another aspect of the invention provides a computer program product comprising computer program code configured, when run a processor which is operatively coupled to an MRI imaging apparatus and a user interface, to cause the processor to perform a method as outlined above, or in accordance with any embodiment described in this disclosure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a processing arrangement and system in accordance with one or more embodiments of the invention; and
Fig. 2 shows an example system in accordance with one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and apparatus for use as part of diagnostic analysis of the psychiatric or neurological health of a patient. Embodiments propose to acquire fMRI data of a particular set of one or more regions of the brain of a patient while the patient is performing a cognitive task, and to record at the same time results of the patient's performance in the cognitive task. A combination of both the cognitive task results and the fMRI data for the brain regions is used to provide an indicator as to a cognitive health status of the patient. More particularly, a normative database of fMRI data results and cognitive task results for a population of patients is used as a reference. This may store results both for healthy patients and non-healthy patients, to provide a normal and abnormal reference.

By way of background, it has been found that poor cognitive task results can be linked with neurological or psychiatric illness. For example, reference is made to the following paper: Russman Block, S., King, A.P., Sripada, R.K., Weissman, D.H., Welsh, R., & Liberzon, I. (2017). Behavioral and neural correlates of disrupted orienting attention in posttraumatic stress disorder. Cognitive, Affective, & Behavioral Neuroscience, 17(2), 422-436.

This paper describes a study using fMRI imaging and the Attention Network Task (to assess attention and executive control). This found that fMRI data for patients suffering from PTSD (post-traumatic stress disorder) exhibited greater resting-state functional connectivity in attentional networks during performance of the Attention Network Task. This information could be used to inform differential diagnosis of patients with respect to PTSD. In particular, if the attention of the patient is impaired, this will present both as a below average performance in the Attention Network Task as well as a deviation from normal brain activation patterns during performance of this task. If the patient's performance on the task is reduced, but they show normal brain activation during the performance of the task, they likely have another underlying deficit that causes reduced performance. In that case, reduced performance on the Attention Network Task may be due to an impairment in cognitive functions or networks other than attention that are also required to perform the task, e.g. executive functions. This is presented simply as an example to illustrate the concept.

More generally, embodiments of the invention propose a method and system for improved detection of cognitive impairment and/or associated brain damage (if any) based on functional neuroimaging and cognitive tests. This approach is based on imaging the particular brain areas and networks that are active during the performance of a specific cognitive test or task. A method and system is proposed which may be used for clinical decision support in neuroimaging diagnosis and treatment. The detection of specific brain damage and associated cognitive impairment is optimized by comparing both cognitive task performance and brain activity in the relevant brain regions to a normative fMRI database. A method and system that integrates functional neuroimaging data and data from cognitive tests is not yet used in clinical practice and would solve a clinical need by aiding in improved accuracy of diagnosis and prediction of progression over time of a wide range of acquired and neurodegenerative brain damage and associated cognitive impairment.

It is also noted that current approached to detection of subtle cognitive impairment and associated brain damage is generic rather than patient specific. In other words, current approaches do not take into account personal specific information. A further aim according to at least one set of embodiment is therefore to provide a method which enables more personalized diagnostic indications, and also prediction of progression of cognitive impairment (and associated risk factors) over time.

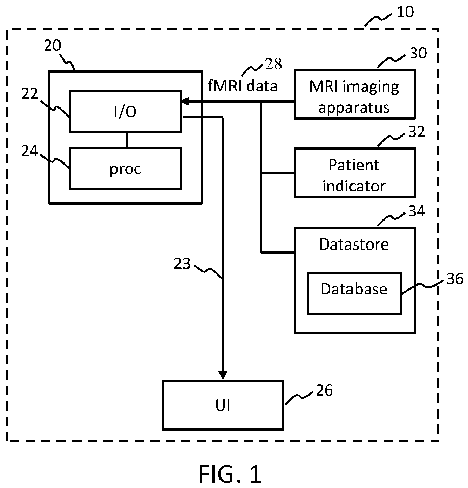

Fig. 1 schematically illustrates an example system 10 which is in accordance with at least one set of embodiments of the invention.

The system 10 comprises a processing arrangement 20. The processing arrangement comprises an input/output 22 and a set of one or more processors 24.

The one or more processors 24 are adapted to implement, in accordance with at least one operation mode, a method comprising the following steps.

The method comprises obtaining functional MRI (fMRI) data 28 for a patient corresponding to one or more pre-determined brain areas of the patient. The one or more brain areas may be chosen so as to be associated with a particular neurological or psychiatric disorder of interest, or alternatively a standard set of one or more brain areas may be chosen. The fMRI data is data acquired while the patient is performing a pre-determined cognitive task. The fMRI data may be, as is illustrated in Fig. 1, obtained from an MRI imaging apparatus 30 being operatively coupled in operation to the input/output 22 of the processing arrangement. In other words, the data is received by the processing arrangement in real time or near real-time with its acquisition. The processing arrangement is shown operatively coupled with the input/output 22 for this purpose. Alternatively, the data might be received from a datastore, where the data was obtained at an earlier time.

As to the acquisition of data for the particular one or more brain locations, this may be set up by an operator of the MRI imaging apparatus, or it may be set up automatically by a control module of the MRI imaging apparatus 30.

The control of the MRI imaging apparatus would most typically be performed by a separate local control module belonging to the MRI imaging apparatus. However, it is also an option for the control of the MRI imaging apparatus to be performed by the processing arrangement 20. For instance, the processing arrangement might be an integral control module which performs both operational control of the MRI apparatus 30 and performs the data processing steps which are part of the main inventive concept being outlined in this disclosure.

With regards to the administering of the cognitive task or cognitive test, this can be performed with a patient indicator subsystem 32 which typically includes a sensory output apparatus with a display mounted within the bore tunnel of the MRI apparatus, and usually headphones for being worn by the subject. The presentation of the task or test prompts to the patient may be controlled by a separate control module instead of (as is schematically illustrated in Fig. 1) being controlled by the processing arrangement 20. It might be controlled by a control module of the MRI imaging apparatus in some cases. The cognitive task or test is administered to the patient simultaneously with acquisition of the fMRI data.

The processing arrangement is further adapted to obtain cognitive task results data for the patient, corresponding to cognitive task administered while acquiring the fMRI data. Administering of the cognitive task comprises presenting a sequence of one or more sensory prompts to the user. It typically further comprises detecting one or more responses from the user. These responses may be indicated directly by the user through a user control device, such as a hand-held control device with one or more buttons for instance. In other cases, the responses might be issued orally by the user, and input manually by an MRI operator using a computer console or other input device. The reference to task results could include the raw response data from the user, and/or could include performance data, by which is meant an indication of success in the test or task, e.g. a number of correct answers, or any other performance metric derived from the responses.

The processing arrangement is further adapted to derive from the obtained fMRI data neurological activity data associated with each of the one or more brain areas. This may comprise for example BOLD data for each of the one or more areas. The activity data may include, for each area, a localized spatial activity mapping, or may simply comprise a single activity metric value for each area. The activity data may comprise an indication of brain activity in each area as a function of time.

The processing arrangement is further adapted to retrieve from a datastore 34 a pre-acquired normative database 36 storing reference neurological activity data and reference cognitive task results data associated with the neurological or psychiatric disorder of interest and associated with the pre-determined cognitive task, for a population of patients. This may comprise statistical summary data pre-computed from activity data and task results data for a population of patients. The statistical data may comprise one or more statistical metrics such as an average, median, inter-quartile range, or any other statistical metric.

The processing arrangement is further adapted to compare the patient's activity data and task results data with the reference activity data and reference task results data.

The processing arrangement is further adapted to provide an output 23 to a user interface 26 based on the comparison. There are different options for the output which is provided, as will be outlined in further detail to follow. The output may simply comprise an indication of a result of the comparison. This alone provides valuable diagnostic support, particularly if the database includes a database portion relating to healthy patients and a second database portion relating to abnormal patients, and wherein the processing arrangement compares the acquired data with both database portions. In other examples, a diagnostic indicator could be generated with further processing based on the comparison, for instance based on an algorithm such as a decision tree algorithm, or based on one or more thresholds pertaining to the comparison results, or based on a machine learning algorithm.

For instance, the processing arrangement may compute a degree of deviation of the patient's neurological activity from an average neurological activity level recorded in the database, and/or a degree of deviation of the patient's cognitive task results from one or more average values recorded in the database. One or more pre-determined thresholds may be applied to one or both of the derived degrees of deviation to determine thereby whether or not the patient has the neurological or psychiatric disorder of interest. As will be described in more detail below, in some embodiments, multiple normative databases can be used, each associated with a different neurological or psychiatric disorder of interest. By determining a degree of deviation of the patient's activity and cognitive tasks results data with the reference data in each database, a determination can be made as to which, if any, of the associated neurological or psychiatric disorders the patient's suffers from.

An aspect of the invention provides just the processing arrangement.

Another aspect of the invention provides a computer-implemented method comprising the above-outlined steps described as being performed by the processing arrangement.

A further aspect of the invention provides system which comprises the processing arrangement 20 and the MRI imaging apparatus 30 operatively coupled with the processing arrangement for acquiring fMRI data of the patient. The system may further comprise the user interface 26. The system may further comprise any other of the components shown in Fig. 1 or described elsewhere in this disclosure.

The system may further include a database storing a plurality of different cognitive tasks or tests for administering to the patient, and from which the system may select. This may be stored in the same datastore 34 as is used to store the database 36 of reference data or in a separate datastore.

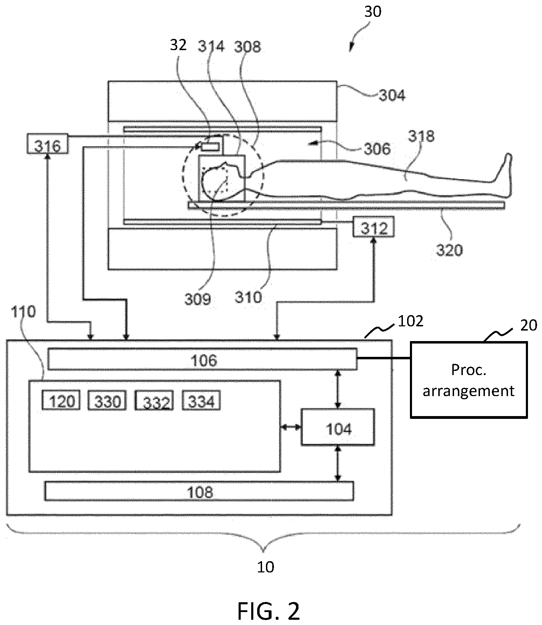

To further explain acquisition of the fMRI data, Fig. 2 shows an example system 10 in accordance with one or more embodiments, in which the MRI imaging apparatus 30 is illustrated in more detail. Fig. 2 illustrates the MRI imaging apparatus 30 operatively coupled with the processing arrangement 20. Other optional components of the system 10 are not shown for simplicity of illustration.

The MRI imaging apparatus 30 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 306 of the cylindrical magnet 304 there is an imaging zone 308 where the magnetic field is strong and uniform enough to perform magnetic resonance imaging. A region of interest 309 is shown within the imaging zone 308. The magnetic resonance data that is acquired typically is acquired for the region of interest. A subject 318 is shown as being supported by a subject support 320 such that at least a portion of the subject 318 is within the imaging zone 308 and the region of interest 309.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 are connected to a magnetic field gradient coil power supply 312. The magnetic field gradient coils 310 are illustrated only schematically. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. In this example the radio-frequency coil 314 is a head coil and the region of interest 309 images the brain of the subject 318.

The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are schematically illustrated only. The radio-frequency coil 314 is intended to also represent a dedicated transmit antenna and a dedicated receive antenna. Likewise the transceiver 316 may also represent a separate transmitter and receiver. The radio-frequency coil 314 may also have multiple receive/transmit elements and the radio frequency transceiver 316 may have multiple receive/transmit channels. For example if a parallel imaging technique such as SENSE is performed, the radio-frequency coil 314 will have multiple coil elements.

Within the bore 306 of the magnet 304 there is a patient indicator 32. The subject indicator may, for example, provide an audio and/or visual stimulus to the subject 318. The patient indicator 32 is able to provide a stimulus or prompt to the user. The patient indicator 32 could, for example, have a light which is visible to the subject 318, be a display, or provide an audio signal. The transceiver 316, the gradient controller 312, and the patient indicator 32 are shown as being connected to a hardware interface 106 of a computer system 102.

The patient indicator 32 is used in administering the cognitive task to the subject.

The patient indictor 32 may further include an input device, e.g. a handheld button or control, permitting the patient to indicate a response as part of a cognitive task.

The medical imaging apparatus is further shown as comprising a computer 102 that comprises a processor 104. The processor is shown as being connected to an optional hardware interface 106, and an optional user interface 108. The user interface 108 may be or include a display for rendering images. The hardware interface 106 may, for example, be a network interface or it may also be used for exchanging data or commands with other components of the medical imaging system.

The processor 104 is further shown as being connected to a memory 110. The memory 110 may be any combination of memory which is accessible to the processor 104. This may include such things as main memory, cached memory, and also non-volatile memory such as flash RAM, hard drives, or other storage devices. In some examples the memory 104 may be considered to be a non-transitory computer-readable medium.

The memory 110 is shown as containing machine-executable instructions 120. The machine-executable instructions 120 enable the processor 104 to perform various data processing tasks and also in some examples to control other components of the medical imaging system 100. The machine-executable instructions 120 enable the processor 104 to perform at least part of the present method.

The memory 110 is further shown as containing pulse sequence commands 330. The pulse sequence commands are either commands or data which can be converted into such commands which enable the processor 104 to control the magnetic resonance imaging system 302. The memory 110 is further shown as containing magnetic resonance imaging data 332 that was acquired by controlling the magnetic resonance imaging system 302 with the pulse sequence commands 330.

Implementation of the system in accordance with preferred sets of embodiments will now be described.

According to a first set of embodiments, which represents a simple case, a single cognitive task is administered to the patient while in the MRI apparatus 30, and fMRI data acquired for the patient during performance of the task. Neurological activity data is derived from the fMRI data for each of one or more pre-determined brain regions that are scanned. A suitable database of reference activity and cognitive task results data is retrieved from the datastore 34 which corresponds to the particular combination of cognitive task administered and neurological or psychiatric disorder which is under investigation. As described above, the reference data in the datastore is used as a comparator, and an output is generated which may be an indication of the results of the comparison or may be a diagnostic indicator obtained based on the comparison and for instance based on use of a diagnostic algorithm or model.

When scanning the patient with the MRI imaging apparatus 30, preferably, in addition to fMRI data, structural MRI data is also obtained for the set of one or more brain regions of interest. Preferably, the reference database 36 further stores reference structural data for the population of patients which can be used as a comparator for the structural MRI data obtained for the new patient.

The obtaining the fMRI data may comprise the processing arrangement 20 generating a control signal for controlling the MRI imaging apparatus 30 to acquire the fMRI data. Alternatively the acquisition of the data may be controlled by a separate control module of the MRI imaging apparatus.

The particular one or more brain areas which are scanned may be selected by the operator of the MRI imaging apparatus 30, or may be selected automatically by the system, for instance based on a lookup table or algorithm, and wherein this may be based on an input from the operator indicative of the particular neurological or psychiatric disorder of interest and/or the cognitive task to be administered. Each disorder is typically associated with particular brain regions that may be affected by the disorder, and also each cognitive task may be associated with particular brain areas that are functional when performing the task, and more particularly with particular neural networks within those areas which are functional when performing the task.

With regards to the cognitive task or test that is administered, a wide variety of cognitive tasks or tests are known in the art, associated with testing the functioning of particular neural networks and type of cognitive activity. In the present case, it may be of particular interest to administer one or more tests which measure attention and executive function, since these cognitive functions are often impaired in patients with subtle cognitive impairment, for example due to a minor traumatic brain injury, ageing or cerebral small vessel diameter.

More generally, the cognitive tests administered may include those for assessing cognitive functioning across a range of different cognitive domains including for instance one or more of: attention, executive functions, memory, perception, motor function, language.

As mentioned above, the system 10 may include a datastore 34 storing a database 36 which holds a plurality of different cognitive tasks or tests for administration to the patient, and from which a selection of one or more can be made.

Each stored cognitive task or test may effectively comprise a computer routine or program represented by a set of computer program code which, when executed by at least one processor of the system, causes a sequence of pre-determined prompts to be communicated to the patient via the patient indicator subsystem 32. These may include any combination of visual prompts, acoustic prompts, haptic prompts or any other sensory prompt. The prompt may ask the patient to perform a particular cognitive function in their mind. Additionally or alternatively, it may ask the patient to provide one or more responses, which might be given for example orally or through control of a user input device.

The responses form the basis of the results of the cognitive task for the patient. Either the raw responses are used as the results of the task, or the responses might be compared with a baseline or reference (e.g. a set of correct answers), and the results are provided by the comparison of the patient's responses with this baseline or reference. The results are recorded, either manually by an operator or automatically.

The cognitive tasks or tests preferably are chosen in advance to have high sensitivity. The cognitive tests may be chosen so as to be compatible and validated for use during neuroimaging. Fine-tuning of the tests could be performed over time based on the particular clinical need and insights derived from research. Therefore, the tests could be editable.

In some examples, the cognitive tasks or tests which are administered to the patient may be selected in part based on one or more demographic classifications of the patient, such as age and education level. Alternatively, rather than varying the cognitive task which is administered based on demographic classification, one or more parameters of a given selected task may be adjusted in dependence upon one or more characteristics of the patient, such age and education level. This provides a form of personalized testing, which can reflect differences in cognitive abilities and/or reflect cognitive deficiencies the patient may have. Thus, in other words, a level of difficulty of a given test may be adjusted based on characteristics of the patient.

In preferred examples, the normative database 36 comprises at least a first database portion which contains reference data associated with a first population of patients who do not suffer from the neurological or psychiatric disorder of interest, and a second database portion which contains reference data associated with a second population of patients who do suffer from the neurological or psychiatric disorder. Although reference is made to database portions, the reference data for the healthy and pathological populations could in fact be stored in separate databases. In other words, for each combination of cognitive task and neurological or psychiatric disorder of interest, there may be: a database or database portion comprising neuroimaging and cognitive test performance data from healthy individuals, and a database or database portion comprising neuroimaging and cognitive test performance data from individuals with brain damage. Both databases may be stratified, e.g. based on age, gender, education level, and/or other demographic factors, and based on diagnosis.

The processing arrangement is adapted to compare the derived activity metric data and obtained task results data with the data in both the first and second database portions.

With regards to the data stored on the, or each, normative database 36, there are different options. There may be stored a plurality of functional (and optionally also structural) neuroimaging data records for a whole population of patients. Alternatively, each database may store one or more statistical metric relating to the whole population of patients, e.g. an average, or interquartile range etc. The type of data stored on the, or each, normative database may be image data. Alternatively, it may be numerical data, indicative for instance of activity level in one or more brain regions, and optionally wherein this is in the form of one or more statistical metrics pertaining to the data, e.g. an average activity level for one or more brain regions, for the population of patients. The neurological activity data may for instance be blood-oxygen-level dependent (BOLD) contrast data. In the latter case, the comparison referred to previously may comprise comparing the derived activity level for each scanned brain region with the average level or range or threshold stored in the database for each brain area. For example, a patient's fMRI data might be considered to match the reference data in the database where his or her BOLD activity is within one standard deviation from the norm (i.e. average) in the database.

The population of patients to which the data in each database corresponds may include patients from a range of different demographic groups (age, gender and education level) and may include also information about the clinical symptoms of the patients.

The, or each, normative database 36, further includes, for the same reference population of patients, data indicative of the results of the cognitive task to which the database corresponds. Again, this may include an individual data record for each patient of the population, or may comprise a statistical metric for the task results for the whole population, or demographically stratified sub-segments of the population.

A second preferred set of embodiments is the same as the first except that, as has been briefly discussed as an option above, the datastore 34 stores a plurality of different normative databases, each corresponding to a different combination of: neurological or psychiatric disorder of interest, and cognitive task or tests administered when acquiring the data.

This set of embodiments permits at least two additional advantageous functionalities. A first is that the operator or the system effectively has a choice as to the particular cognitive task which is administered for testing a particular neurological or psychiatric disorder, and also has a choice from a number of different neurological or psychiatric disorders to test for. Thus, the system and method in this set of embodiments permits a wider of range of options for testing the subject and analyzing their cognitive health. A second additional functionality, following from the first, is that the system or the operator may elect to administer multiple different cognitive tests (and acquire a corresponding fMRI dataset for each) as part of testing for a single given neurological or psychiatric disorder, and/or may elect to test for multiple different neurological or psychiatric disorders. In the latter case, the system may implement a sequence of tests (known as a battery of tests), which include tests associated with different neurological or psychiatric disorders, as a means of assisting a differential diagnosis between the different neurological or psychiatric disorders.

Thus, in this set of embodiments, the datastore stores a plurality of different normative databases, each respectively storing reference activity data and reference cognitive task results data associated with a different specific combination of a neurological disorder of interest and a cognitive task administered.

The processing arrangement 20 may query the database according to the specific neurological disorder and cognitive task to which the newly obtained results data corresponds.

In use, in accordance with this second set of embodiments, where only a single cognitive task is to be administered, or at least only one at a time, the process implemented may, in summary, run as follows.

The process may comprise:
selecting (e.g. an operator selecting or a computer program selecting) a disorder of interest from a pre-determined set of different disorders;
further selecting, for the selected disorder, one of a set of one or more cognitive tests/tasks which are associated with the selected disorder;
identifying a set of one or more brain regions of interest associated with the disorder;
acquiring fMRI data associated with those brain regions while administering the selected test/task to the patient;
acquiring simultaneously with the acquiring of the fMRI data results of the cognitive task based on subject indications during the task; and
retrieving from the datastore a database storing statistical results for neural activity and cognitive task results for the selected disorder and the selected cognitive task;
deriving from the obtained fMRI data neurological activity data associated with each of the one or more brain areas;
comparing the patient's activity data and task results data with the reference activity data and reference task results data.

The above process may be repeated a number of times, for each of a plurality of different neurological or psychiatric disorders of interest. From this, a differential diagnostic indicator could be derived between the different neurological or psychiatric disorders of interest.

In a case where a plurality of different tests are to be administered (i.e. a battery of tests), the process followed, in summary, may be as follows. The processing arrangement may in this case be adapted to:
obtain a plurality of fMRI data sets for the patient, each acquired while a different cognitive task is administered to the patient,
obtain cognitive task results for the patient for each administered cognitive task;
retrieve from the datastore a normative database associated with each different cognitive task, and further associated with the neurological or psychiatric disorder;
compare, for each cognitive task, activity data derived from the corresponding fMRI data set with reference activity data in the corresponding retrieved database, and comparing the cognitive task results with the reference cognitive task results in said corresponding database.

The plurality of cognitive tests may all relate to the same neurological or psychiatric disorder of interest or different disorders of interest.

The method may further comprise providing a differential diagnostic indicator based on the above process.

In some embodiments, where multiple cognitive tests are to be administered to the patient, each new test to be administered may be selected by processing arrangement 20 based on performance on previously administered cognitive tests and associated neuroimaging results. This can be done after assessments (or immediately in real-time if possible) based on cognitive test performance and brain activation patterns. For instance, if a patient shows normal performance on a test assessing attention, but abnormal brain activation patterns, this may indicate that he uses different cognitive functions to perform the cognitive test suggesting compensation of impaired cognitive functions with non-impaired cognitive functions. In this case, it may be advised to conduct additional cognitive tests that assess the cognitive functions used to perform the attention test as evident from the brain activation pattern.

According to at least some examples, the processing arrangement 20 is adapted to: apply a selection algorithm to determine a new cognitive test to be administered to the patient in dependence upon the obtained cognitive task results and/or in dependence upon the obtained neurological activity data. The processing arrangement may be further adapted to retrieve from the datastore a normative database containing reference activity data and reference cognitive task results data associated with the selected cognitive task. The processing arrangement may be further adapted to generate control signals for controlling a patient interface control module (for example a control module of the patient indicator subsystem 32 mentioned above) to administer the selected cognitive task and for controlling an MRI imaging apparatus to obtain second fMRI data of the patient while the selected cognitive task is being administered.

The selection algorithm may for instance be a pre-determined decision tree algorithm, wherein a result of one cognitive task and/or associated neurological activity data is used by the algorithm to determine a next task to be administered in a sequential hierarchical decision structure.

In accordance with any of the above described embodiments, it is preferable that the data in the, or each, normative database 36 are stratified based on one or more demographic classifications of the population of patients to whom the data pertain. These demographic classifications may include for instance age, gender, occupation, education level, and/or other demographic factors. The data may be further stratified based on further characteristics such as factors relating to medical history, or lifestyle habits (e.g. drinking, smoking).

The processing arrangement may in this case be further adapted to obtain an indication of the one or more demographic classifications for the patient; and to filter the normative database according to the demographic classification of the patient before performing the comparison.

Thus, comparison is made between similar groups of patients accounting for gender, age, and other predefined features.

As mentioned above, the neural activity data and cognitive task results data may be compared to both a database portion corresponding to healthy individuals and to the database portion corresponding to individuals with a specific impairment. Multimodal information may be stored about each individual in the normative database. Comparisons can be conducted with stratified samples as described above.

In accordance with either of the above described preferred sets of embodiments, the method comprises generating an output for a user interface based on the comparison of the activity data and cognitive task results data with the reference data in each of the one or more databases 36. There are different options for the output which is generated and how this is derived.

In some examples, the output to the user interface 26 comprises simply a representation of a result of the comparison. For example, results are presented on the user interface 26 showing the performance on cognitive tests compared to the normative sample represented in the normative database 36, optionally corrected for relevant characteristics, e.g. age and education level, and also showing the neural activity in different brain regions compared to the normative sample represented in the database.

In further examples, additional processing may be performed to determine a diagnostic indicator for one or more neurological or psychiatric disorders based on the comparison, and to provide the diagnostic indicator as the output to the user interface.

In some examples, the results of the comparison may be processed with a diagnostic algorithm or model to determine one or more of: a neurological or psychiatric disorder of the patient, a type and location of cognitive impairment in the brain, a type and location of any brain injury.

In some examples, the results of the comparison may be processed with an algorithm to generate an assessment of a patient's cognitive functioning and brain damage. The assessment includes which cognitive domains and which brain areas and networks are impaired and will also include an assessment of severity of the impairment. The assessment may additionally include also an indication of one or more compensation mechanisms that a patient may use to work around any deficiencies identified.

The above-referenced user-interface 26, to which the generated output is communicated, may comprise one or more sensory output components. These may include one or more of: a display device, an acoustic output device, a haptic output device. The user interface may further include one or more user input components, such as a keypad and/or a pointer device. The user interface may comprise a touchscreen display in some embodiments.

The provided output to the user interface 26 may comprise an indication of a result of the previously discussed comparison performed between the patient's data and the reference data in the database 36.

In preferred embodiments, the fMRI imaging data acquired for the patient is presented on a display of the user interface 26 in real time. Preferably also a visual representation of the derived neurological activity data is also presented on the display, for instance overlaid on the fMRI imaging data, or provided as part of the fMRI imaging data.

In preferred embodiments, a visual representation of a comparison between the patient's neurological activity in the one or more scanned brain regions, and the activity represented in the normative database 36 may be provided on the display. For instance, two images could be presented side-by-side on the screen, one representing a visual map or depiction of the neurological activity in the patient's brain as a function of position and/or time, and one representing a visual map or depiction of an average neurological activity as a function of position and/or time for the population of patients represented in the database 36. The average image could be an average for a sub-stratum of the population, filtered according to a demographic classification of the patient, as discussed above. The display may furthermore show images of average neurological activity both for a healthy population and a population which has been diagnosed with the neurological or psychiatric disorder of interest. Thus, the operator can easily compare the patient's result with the that of the healthy and non-healthy populations.

As well as presenting a comparison of the neurological activity, the user interface 26 also presents a computed comparison results, e.g. indicating a deviation of the patient's activity levels from an average of the healthy population, and/or from an average of the non-healthy population. This can be provided in the form of a numerical indicator of the disparity, e.g. a number of standard deviations away from the mean in each case.

As well as presenting a comparison of the neurological activity results, the user interface 26 preferably also provides an indication of a comparison of the cognitive task results of the patient with an average of results for the same task for the population of patients in the database 36. Again, preferably, this comparison is performed, and the result presented, for a population of both healthy and non-healthy individuals, and specifically for a substratum of the database 36 population which matches the patient's demographic classification.

Where, as described above, multiple cognitive tests are administered (for the same and/or different neurological or psychiatric disorders), and data obtained for the patient for each one, the above-described comparison results may be presented for each one in turn. A GUI may be provided on a display of the user interface 26 permitting the operator to switch between the different sets of results and review each one at will. It may also permit the operator to select to make other relevant comparisons, e.g. with patient groups with a different diagnosis to the patient.

In accordance with any of the above-described embodiments, in preferred examples, each newly acquired dataset of fMRI data and cognitive tasks results may be used to update the normative database that is being used as the comparator. For example, this may comprise simply appending the activity data derived from the fMRI scan data, along with the cognitive tasks results data, to the normative database 36. In other cases, it may comprise updating a statistical metric stored in the database based on the new data, e.g. updating an average value for the activity level in each brain area based on the newly acquired data.

Thus, in this set of embodiments, the normative data will be updated over time. This means that the normative database will grow over time as more individuals are scanned. This will ensure greater representativeness of the normative database.

In accordance with any of the above described embodiments, it is advantageous to include functionality for predicting progression of a patient's condition. By way of example, in accordance with one or more embodiments, a multimodal (or multifactorial) assessment may be performed based on application of a progression algorithm, to predict progression of cognitive impairment over time. The prediction may be based on performance on cognitive tests, neuroimaging findings, and other disease-specific data.

For example, in the domain of traumatic brain injury (TBI), prediction may be based on one or more of: pre-injury physical and psychiatric comorbidities, early post-concussive symptoms, early post-traumatic stress, headache at ED, nausea/vomiting at ED, neck pain at ED, emotional distress at 2 weeks, maladaptive coping at 2 weeks, intoxication at time of injury, type of injury, age, education level, cause of injury, and score on the Glasgow Coma Scale.

In the domain of neurodegenerative diseases, multimodal assessment could be based on one or more of: performance on cognitive tests, neuroimaging findings, patient characteristics such as age, gender and education level, lifestyle factors such as level of physical activity and smoking, genetic testing, and blood testing, cerebrospinal fluid, and others.

The progression prediction itself may be performed for example using a statistical prediction model such as a pre-trained machine learning algorithm. The machine learning algorithm may be trained using a training database comprising data records for a plurality of patients.

In accordance with any of the above-described embodiments, the described data analysis steps may preferably be done in real time. In particular, deriving the neurological activity data, comparing the activity data and task results data with the reference data, and deriving the output based on the comparison may be done in real time with acquisition of the fMRI data. This enables the results to be presented to the operator of the system on the user interface 26 simultaneously with the scanning of the patient. A benefit of this real-time analysis is that it allows for the parameters of the examination to be adjusted based on the results. For example, the real-time analysis could be used by the operator to fine-tune the cognitive tests, for example to adjust the difficulty level and/or to adjust technical parameters in order to prevent the use of straightforward compensation mechanisms by the patient.

To further illustrate the concept of the invention, three example neurological or psychiatric disorders are outlined below to which embodiments of the invention might advantageously be applied. These examples are purely illustrative and non-limiting.

A first example application is for the assessment of depression stress. Reference is made to the paper: Yan et al. (2019), Reduced default mode network functional connectivity in patients with recurrent major depressive disorder. PNAS, 116, 9078-9083. This outlines a study involving fMRI data acquired from 848 patients with Major Depressive Disorder (MDD) and 794 normal control patients. In the MDD cohort, the fMRI data showed decreased functional connectivity in the default mode network, confirming a key role of the default mode network in MDD. The decreased functional connectivity in the default mode network was positively related to symptom severity in recurrent MDD. Thus, if the psychiatric disorder of interest is depression, the cognitive task administered may be one which tests the default model functional network.

A second example application is for the assessment of claustrophobia. Reference is made to the paper: Feng, Zheng & Feng (2015). Spontaneous brain activity following fear reminder of fear conditioning by using resting-state functional MRI. Scientific reports, 5, 16701. This outlines a study which examined spontaneous neuronal activity following a fear (such as claustrophobia) memory activation. It reported significant enhanced functional connectivity between the amygdala and ventromedial prefrontal cortex upon fear memory activation. Thus if the psychiatric disorder of interest is claustrophobia, the cognitive task administered to the patient may be one which prompts the user to recall a fear memory, and wherein the brain areas which are scanned include the region between the amygdala and ventromedial prefrontal cortex.

A third example application is for the assessment of task-associated stress, which can be relevant for a range of neurological or psychiatric disorders, including for instance mild traumatic brain injury. During sustained periods of a demanding cognitive workload, entailing cognitive fatigue, humans typically display time-on-task effects, in which performance steadily deteriorates over a period of task engagement. Reference is made to the following paper: Lim et al. (2010). Imaging brain fatigue from sustained mental workload: an ASL perfusion study of the time-on-task effect. Neuroimage, 49, 3426-3435. This paper reported persistent effects of cognitive fatigue in the fronto-parietal network after a period of heavy mental work and indicate the critical role of this attentional network in mediating time-on-task effects.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processing arrangement (20) comprising one or more processors (24) adapted to:
obtain functional MRI (fMRI) data for a patient corresponding to one or more areas of the brain, wherein the fMRI data is acquired while the patient is performing a pre-determined cognitive task;
obtain cognitive task results data for the patient, corresponding to the cognitive task administered while acquiring the fMRI data;
derive from the obtained fMRI data neurological activity data;
retrieve from a datastore (34) a pre-acquired database (36) storing reference neurological activity data and reference cognitive task results data associated with a neurological or psychiatric disorder of interest and associated with the pre-determined cognitive task, for a population of people;
compare the patient's activity data and task results data with the reference activity data and reference task results data; and
provide an output (23) to a user interface (26) based on the comparison.

2. The processing arrangement of claim 1, wherein the processing arrangement is further adapted to derive a diagnostic indicator for the neurological or psychiatric disorder of interest based on the comparison, and to provide the diagnostic indicator as the output to the user interface.

3. The processing arrangement of claim 1 or 2, wherein the output to the user interface is indicative of the results of the comparison.

4. The processing arrangement of any of claims 1-3, wherein the neurological activity data is blood-oxygen-level dependent (BOLD) contrast data.

5. The processing arrangement of any of claims 1-4, wherein the datastore stores a plurality of different databases, each respectively storing reference activity data and reference cognitive task results data associated with a different specific combination of a neurological disorder of interest and a cognitive task administered.

6. The processing arrangement of claim 5, wherein the processing arrangement is adapted to:
obtain a plurality of fMRI data sets for the patient, each acquired while a different cognitive task is administered to the patient,
obtain cognitive task results for the patient for each administered cognitive task;
retrieve from the datastore a database associated with each different cognitive task, and further associated with the neurological or psychiatric disorder of interest;
compare, for each cognitive task, activity data derived from the corresponding fMRI data set with reference activity data in the corresponding retrieved database, and comparing the cognitive task results with the reference cognitive task results in said corresponding database.

7. The processing arrangement of any of claims 1-6, wherein the database comprises at least a first database portion which contains data associated with a first population of people who do not suffer from the neurological or psychiatric disorder of interest, and a second database portion which contains data associated with a second population of people who do suffer from the neurological or psychiatric disorder.

8. The processing arrangement of any of claims 1-7, wherein the reference activity data and reference task results data comprise statistical data pre-computed from activity data and task results data for a population of people.

9. The processing arrangement of any of claims 1-8, wherein the processing arrangement is adapted to update the database based on the obtained activity data for the patient, and the task results data for the patient.

10. The processing arrangement of any of claim 1-9, wherein the data in the database are stratified based on one or more demographic classifications of the population of people to whom the data pertain.

11. The processing arrangement of claim 10,
wherein the processing arrangement is further adapted to obtain an indication of the one or more demographic classifications for the patient; and
wherein the processing arrangement is adapted to filter the database according to the demographic classification of the patient before performing the comparison.

12. The processing arrangement of any of claims 1-11, wherein the processing arrangement is further adapted to:
apply a selection algorithm to determine a new cognitive test to be administered to the patient in dependence upon the obtained cognitive task results and/or in dependence upon the obtained neurological activity data;
retrieve from the datastore a database containing reference activity data and reference cognitive task results data associated with the selected cognitive task;
generate control signals for controlling a patient interface control module to administer the selected cognitive task and for controlling an MRI imaging apparatus to obtain second fMRI data of the patient while the selected cognitive task is being administered.

13. The processing arrangement of any of claims 1-12, wherein the obtaining the fMRI data comprises the processing arrangement generating a control signal for controlling an MRI imaging apparatus to acquire the fMRI data.

14. A system (10) comprising:
the processing arrangement (20) of any of claims 1-13; and
an MRI imaging apparatus (30) operatively coupled with the processing arrangement for acquiring fMRI data of the patient.

15. A computer program product comprising computer program code configured, when run a processor which is operatively coupled to an MRI imaging apparatus and a user interface, to cause the processor to perform a method comprising:
obtaining functional MRI (fMRI) data for a patient corresponding to one or more areas of the brain, wherein the fMRI data is acquired while the patient is performing a pre-determined cognitive task;
obtaining cognitive task results data for the patient, corresponding to the cognitive task administered while acquiring the fMRI data;
deriving from the acquired fMRI data neurological activity data;
retrieving from a datastore (34) a pre-acquired database (36) storing reference neurological activity data and reference cognitive task results data associated with a neurological or psychiatric disorder of interest and with the pre-determined cognitive task, for a population of people;
comparing the patient's activity data and cognitive task results data with the reference activity data and reference cognitive task results data; and
providing an output (23) to a user interface (26) based on the comparison.
